# EUROPEAN PATENT SPECIFICATION

(11) **EP 2 451 407 B1**
(45) Date of publication and mention of the grant of the patent: **21.10.2015**
(21) Application number: 10797406.5
(22) Date of filing: 12.07.2010
(51) Int. Cl.: A61F 2/46, A61F 2/30

(54) **APPARATUS FOR TREATMENT OF ARTHROSIS OR OSTEOARTHRITIS IN A JOINT OF A MAMMAL OR HUMAN PATIENT**
VORRICHTUNG ZUR BEHANDLUNG VON ARTHROSE ODER OSTEOARTHRITIS IN EINEM GELENK EINES SÄUGETIERS ODER EINES MENSCHLICHEN PATIENTEN
DISPOSITIF DE TRAITEMENT DE L'ARTHROSE OU D'UNE OSTÉOARTHROSE DANS UNE ARTICULATION DE MAMMIFÈRE OU DE PATIENT

(30) Priority: 10.07.2009 SE 0900981; 10.07.2009 SE 0900957; 10.07.2009 SE 0900959; 10.07.2009 SE 0900960; 10.07.2009 SE 0900962; 10.07.2009 SE 0900963; 10.07.2009 SE 0900965; 10.07.2009 SE 0900966; 10.07.2009 SE 0900968; 10.07.2009 SE 0900969; 10.07.2009 SE 0900970; 10.07.2009 SE 0900972; 10.07.2009 SE 0900973; 10.07.2009 SE 0900974; 10.07.2009 SE 0900976; 10.07.2009 SE 0900978; 10.07.2009 SE 0900958; 10.07.2009 SE 0900961; 10.07.2009 SE 0900964; 10.07.2009 SE 0900967; 10.07.2009 SE 0900971; 10.07.2009 SE 0900975; 10.07.2009 SE 0900977; 10.07.2009 SE 0900979; 10.07.2009 SE 0900980; 30.07.2009 US 229752 P; 30.07.2009 US 229755 P; 30.07.2009 US 229761 P; 30.07.2009 US 229767 P; 30.07.2009 US 229778 P; 30.07.2009 US 229786 P; 30.07.2009 US 229789 P; 30.07.2009 US 229796 P; 30.07.2009 US 229735 P; 30.07.2009 US 229738 P; 30.07.2009 US 229739 P; 30.07.2009 US 229743 P; 30.07.2009 US 229745 P; 30.07.2009 US 229746 P; 30.07.2009 US 229747 P; 30.07.2009 US 229748 P; 30.07.2009 US 229751 P; 30.07.2009 US 229730 P; 30.07.2009 US 229731 P; 30.07.2009 US 229733 P; 30.07.2009 US 229802 P; 30.07.2009 US 229805 P; 30.07.2009 US 229811 P; 30.07.2009 US 229815 P; 30.07.2009 US 229816 P; 24.11.2009 WO PCT/SE2009/000502
(43) Date of publication of application: 16.05.2012
(73) Proprietor: Kirk Promotion LTD., 114 79 Stockholm (SE)
(72) Inventor: FORSELL, Peter, 6300 Zug (CH)
(86) International application number: PCT/SE2010/050818
(87) International publication number: WO 2011/005199

(56) References cited:
- WO-A1-01/87195
- WO-A1-2009/143646
- US-A1- 2004 236 424
- US-A1- 2004 236 424
- US-A1- 2004 247 641
- US-A1- 2006 190 078
- US-A1- 2009 043 344
- US-A1- 2009 076 605
- US-B1- 6 306 177
- US-B1- 6 306 177

## Description

### BACKGROUND

The present invention relates generally to surgical treatment of joints. More particularly the invention relates to an apparatus and methods for 5 treatment of arthrosis or osteoarthritis in a joint of a mammal or human patient.

Traditionally, surgical treatment of damaged joint surfaces (for example in the hip or knee joint) has implied a relatively complicated procedure. For example, in case of arthrosis in the hip joint, a substantial portion of the femoral bone is normally replaced with a prosthesis. This type of operation affects comparatively large muscles and/or many ligaments and tendons, which in turn, leads to a long period of convalescence for the patient. Moreover, if only the cartilaginous tissue of the joint is damaged, it is actually unnecessary to remove the healthy femoral bone tissue.

In the international patent application published as WO 01/87195 an instrument for injecting a material replacing an intervertebral disk is disclosed. This prior art does not describe the use of the device with a biocompatible material for replacing damaged bone of a worn out joint surface wherein the biocompatible material is configured to assume a solid form under predefined conditions, and when in the solid form having a resistance to wear.

### SUMMARY

The object is therefore to alleviate the above problems and provide an uncomplicated solution for treating arthrosis in joints, which minimizes the removal of healthy tissue and decreases the period of convalescence and the pain to the area of the joint.

According to one aspect of the invention, a system comprises a biomaterial for replacing damaged bone of a worn out joint surface and an apparatus for treatment comprising a reservoir configured to hold a volume of a biocompatible material in liquid form having a temperature higher than 100°C outside of a body containing a joint to be treated, and an instrument having a general tube shape, a distal end of the instrument being configured to be inserted into the joint, a proximal end of the instrument being connected to the reservoir and configured to receive the liquid material there from, the instrument being configured to feed the liquid material having a temperature higher than 100°C from the proximal end to the distal end for deposition on at least one damaged surface of the joint, such that adjacent nerves are damaged by the heat of the material, the material being configured to assume a solid form under predefined conditions, and when in the solid form the material having a resistance to wear adapted to replace a worn out joint surface. According to one embodiment, the apparatus further includes a flexible and collapsible mould member. This member has a pre-produced shape adapted to a shape and size of at least one of the at least one damaged surface. Thus, the mould member may contact and cover this joint surface. Additionally, the mould member is configured to be inserted into the joint, and be form-fitted to said damaged surface. The mould member is also configured to be connected to the distal end of the instrument, and when placed in the joint (J) receive the material in liquid form being fed through the instrument. The mould member has an internal volume configured to be filled with liquid material received via the instrument. Moreover, the mould member is configured to enable the predefined conditions for accomplishing a transition from the liquid form to the solid form after that the mould member has been filled with the liquid material.

The mould member is advantageous, since it facilitates targeting a predetermined volume of the liquid material to a specific joint surface both in terms of thickness and lateral coverage.

In any of the embodiments the material could comprise at least one material selected from the group consisting of: polytetrafluoroethylene, perfluoroalkoxy, fluorinated ethylene propylene, polyethylene, and acrylic polymer mixed with alumina trihydrate.

One advantage is that very small incisions is required. Thus, the healing process after the surgery can be made relatively short. Moreover, no healthy bone tissue is removed unnecessarily. Further advantages, beneficial features and applications will be apparent from the following description and the dependent claims.

### BRIEF DESCRIPTION OF THE DRAWINGS

Embodiments are now to be explained more closely, which are disclosed as examples, and with reference to the attached drawings.
- Figure 1: schematically illustrates an apparatus according to one embodiment;
- Figure 2: illustrates how the hip joint of a patient may be reached according to two embodiments;
- Figure 3: schematically illustrates how a mould member adapted to a first joint surface is connected to the proposed apparatus according to a first embodiment;
- Figure 4: schematically illustrates how a mould member adapted to a second joint surface is connected to the proposed apparatus according to a second embodiment;
- Figure 5: illustrates how the knee joint of a patient may be treated according to one embodiment;
- Figure 6: illustrates how laparoscopic/arthroscopic trocars are approaching the hip joint from the abdominal side of the pelvic bone;
- Figure 7: illustrates how a hole is being created in the pelvic bone from the abdominal side thereof;
- Figure 8: illustrates how a small hole is being created in the pelvic bone from the abdominal side thereof;
- Figure 9: illustrates how a hole is being created in the femoral bone from the lateral side of the patient;
- Figure 10: illustrates how a hole has been created in the hip joint capsule.
- Figure 11a: shows a mould placing instrument.
- Figure 11b: shows a mould placing instrument.
- Figure 11c: shows a mould placing instrument;
- Figure 12: shows the insertion of a mould;
- Figure 13a: shows the insertion of a mould;
- Figure 13b: shows the insertion of a mould;
- Figure 13c: shows the filling of a mould;
- Figure 13d: shows the hip joint in section after the mould has been filled;
- Figure 14: shows the placing of a mould in the knee joint;
- Figure 15: shows the placing of a mould in the knee joint;
- Figure 16: shows the filling of a mould in the hip joint;
- Figure 17: shows the filling of a mould in the hip joint;
- Figure 18: shows the filling of a mould in the hip joint;
- Figure 19: shows the hip joint in section after the mould has been filled;
- Figure 20: shows a medical device for injecting a fluid.

### DETAILED DESCRIPTION

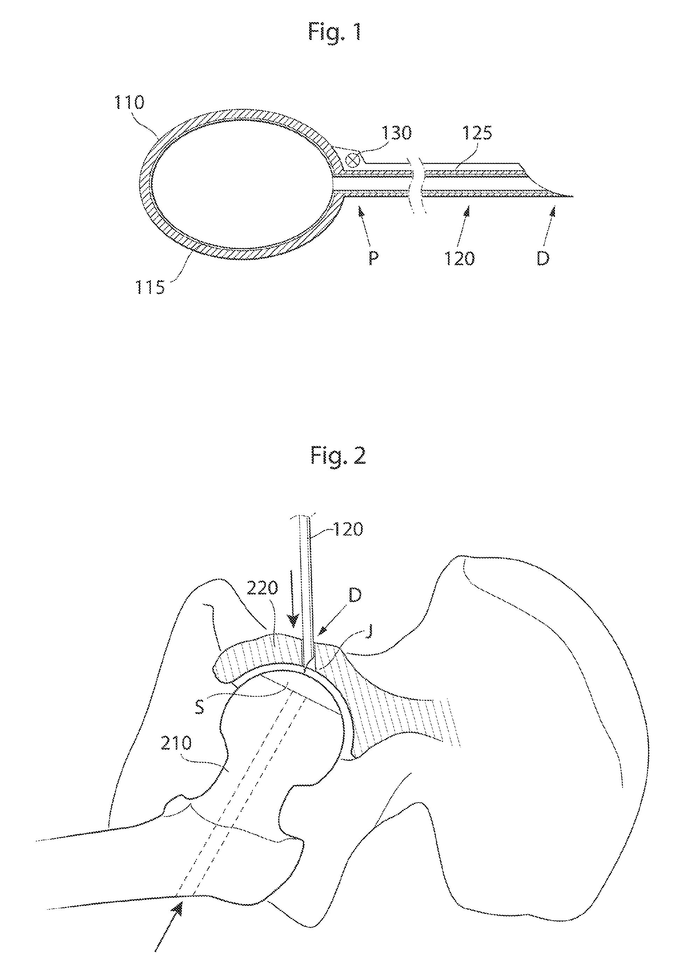

We refer initially to Figure 1, which schematically illustrates an apparatus for treatment of arthrosis in a joint according to one embodiment. Typically, the joint is included in a human patient. However, technically, any other mammal may equally well be treated by means of this apparatus, e.g. a horse.

The apparatus includes a reservoir 110 and an instrument 120. The reservoir 110 is configured to hold a volume of a biocompatible material in liquid form outside of a body containing a joint to be treated. The biocompatible material in the reservoir 110 is initially liquid. However, under predefined conditions the material is configured to assume a solid form. For instance, the material may be liquid when its temperature is above a certain level, and solid otherwise; or it may be liquid until it is exposed to a specific type of radiation, say ultraviolet light. These aspects will be elaborated below. In any case, when in the solid form the material has a resistance to wear adapted to replace a worn out joint surface.

The instrument 120 has a general tube shape, which may be substantially more elongated than what is apparent from the examples shown in Figure 1. The instrument 120 may also be articulated (i.e. including one or more links), so as to enable a more convenient access to joint surfaces inside the body. Irrespective of the length and specific design of the instrument 120, a distal end D thereof 120 is configured to be inserted into the joint to be treated. A proximal end P of the instrument 120 is connected to the reservoir 110 and configured to receive the material in liquid form from the reservoir 110. The instrument 120 is further configured to feed the liquid material from the proximal end P to the distal end D, such that the material can be deposed on at least one damaged joint surface. This instrument could also be inserted into the joint via an arthroscopic trocar i.e. inside another tube, wherein the end of the instrument could be more flat.

According to one embodiment, it is presumed that the biocompatible material in the reservoir 110 is liquid because it has an elevated temperature (i.e. above 37 degrees Celsius), say 50, 70, 90, 100, 150, 200, 300 or 400 degrees Celsius, or any other temperature within an interval from 37 to 500 degrees Celsius. Therefore, the reservoir 110 is configured to hold the biocompatible material at the temperature in question, i.e. 50, 70, 90, 100, 150, 200, 300, 400 degrees Celsius or more. To this aim, the reservoir 110 preferably includes at least one shield member 115, which is configured to isolate the reservoir 110 from the patient's body. Naturally, if the biocompatible material in the reservoir 110 is hot, the material will be hot also when passing through the instrument 120. Hence, the instrument 120 preferably likewise includes at least one shield member 125 configured to isolate the body from liquid material. The material being injected at a high temperature will damage the adjacent nerves, thereby reducing the pain to the area of the joint. Suitable biocompatible materials being liquid at an elevated temperature, and that become solid when cooling off, may comprise fluoropolymers, e.g. in the form of polytetrafluoroethylene, perfluoroalkoxy and/or fluorinated ethylene propylene.

According to another embodiment, it is presumed that the biocompatible material in the reservoir 110 contains two different components. Each of these components is liquid when isolated from the other component. However, when the components are mixed in predefined proportions they develop a solid material. Hence, the two components may represent a hardenable component and a hardening agent respectively. In this embodiment, the reservoir 110 is configured to hold the two components separated from one another during an initial phase of a treatment. During a surgery phase subsequent to the initial phase, the reservoir 110 is configured to mix the two components into a mix. Here, the components are mixed in such proportions that the mix remains liquid during a period required to feed the mix through the instrument 120 to the at least one damaged joint surface in the joint to be treated. Moreover, the instrument 120 is configured to enable such a conveying of the mix.

According to another embodiment, it is presumed that the biocompatible material in the reservoir 110 is liquid due to the fact that the material has not yet been exposed to a specific type of radiation. For example, the material is configured to be liquid if it has been exposed to electromagnetic radiation whose intensity in a predefined spectrum is below a first predefined energy level per unit volume; and the material is configured to be solid if it has been exposed to electromagnetic radiation whose intensity in the predefined spectrum is above a second predefined energy level per unit volume. Here, the apparatus includes an electromagnetic radiation source, which is configured to convey electromagnetic radiation in the predefined spectrum to the at least one damaged joint surface via the instrument 120. Thus, by activating the radiation source after deposition of biocompatible material on the joint surface, the material may be caused to transition from the liquid form to a solid form.

According to another embodiment, it is presumed that the biocompatible material in the reservoir 110 is liquid due to the fact that the material has not yet been exposed to a specific type of mechanical energy. For example, the biocompatible material may be configured to be liquid when it has been exposed to ultrasonic energy in a predefined spectrum whose intensity is below a first predefined energy level per unit volume; and be solid when it has been exposed to ultrasonic energy in the predefined spectrum above a second predefined energy level per unit volume. Here, the apparatus includes an ultrasound source configured to convey ultrasonic energy in the predefined spectrum to the at least one damaged joint surface via the instrument 120. Thus, by activating the ultrasound source after deposition of biocompatible material on the joint surface, the material may be caused to transition from the liquid form to a solid form.

Preferably, since the biocompatible material will be deposed in a patient's body, the reservoir 110 and the connection between the reservoir 110 and the instrument 120 are configured to maintain the material sterile throughout the entire procedure.

Furthermore, according to all aspects, it is advantageous if the apparatus includes a light source 130 configured to illuminate the joint being treated during deposition of the liquid material on at least one damaged surface of this joint.

Figure 2 illustrates how a damaged surface S of the hip joint J of a patient is reached according to two embodiments. In both cases the joint J is accessed through at least one bone of the body, namely either by passing via the femoral bone 210 or the pelvis bone 220

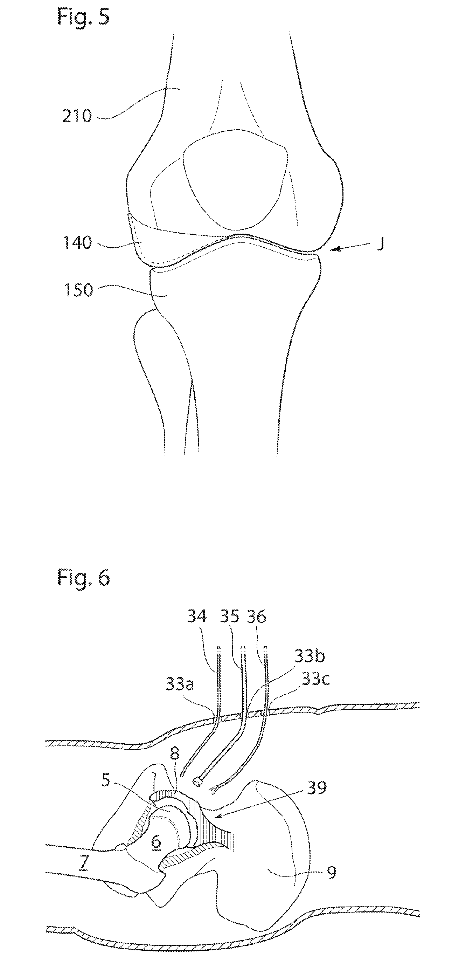

For example, the distal end D of the proposed instrument 120 may be inserted into the hip joint J by passing via the pelvis bone 220 from inside the abdomen, as illustrated to the right. Here, for reasons of clarity, Figure 2 only shows the distal-most end D of the instrument 120 as a straight segment. However, of course, in order to reach the joint J, the instrument 120 may be provided with one or more links (not shown). According to one aspect, the apparatus is surgically placed in the patient via a laparoscopic abdominal approach, shown in fig 6. Specifically, this involves inserting the tube-shaped instrument 120 into the abdomen of the patient's body. Gas is then supplied into the patient's abdomen via the instrument 120, so as to expand the abdominal cavity. At least two laparoscopic trocars are placed in the patient's body, and a camera is inserted through one of the laparoscopic trocars into the patient's abdomen. Moreover, at least one dissecting tool is inserted through one of the at least two laparoscopic trocars. The method further involves dissecting a bone area opposite to the hip region, and drilling at least one hole in the bone of the patient from the abdomen reaching the hip joint, e.g. as illustrated in Figure 2. Surgery and treatment for treating arthrosis or osteoarthritis of the hip joint J is then performed through this hole by using the proposed apparatus.

Alternatively, the distal end D of the proposed instrument 120 may be inserted into the hip joint J by passing via the femoral bone 210 of the body, as illustrated to the left in Figure 2. According to one aspect, the apparatus is surgically placed in the patient via a femoral bone approach, which involves the following. The patient's skin is cut at the lateral upper femoral region. Then, at least one hole is drilled in the bone of the patient through the upper femoral region reaching the hip joint J. Finally, surgery and treatment is performed through this at least one hole for treating arthrosis or osteoarthritis of the hip joint J by using the proposed apparatus.

Preferably, the step of drilling the at least one hole in the bone involves drilling the hole in such a way that a plug of bone is detached into the abdomen. Prior to completing the operation, the method further comprises replacing the plug.

The instrument 120 may also be configured to be inserted into the joint J by passing via a capsula of the joint J.

Irrespective of how the joint J is accessed for treatment of arthrosis therein, according to these embodiments, the method involves the following. A volume of a biocompatible material in liquid form is fed into the reservoir 110 of the apparatus. The reservoir 110 is located outside of a body containing a joint J to be treated. Then, the distal end D of the instrument 120 is inserted into the joint J, and the proximal end P of the instrument 120 is connected to the reservoir 110. Subsequently, the liquid material from the reservoir 110 is received in the instrument 120. Thereafter, the liquid material is fed from the proximal end P to the distal end D of the instrument 120, such that the material is deposed on at least one damaged surface S of the joint J. Finally, the material is caused to transition from the liquid form to a solid form. When in the solid form, the biocompatible material has a resistance to wear adapted to replace a worn out joint surface. Namely, the material is configured to assume the solid form under predefined conditions, for instance in response to a temperature drop, or exposure to radiation.

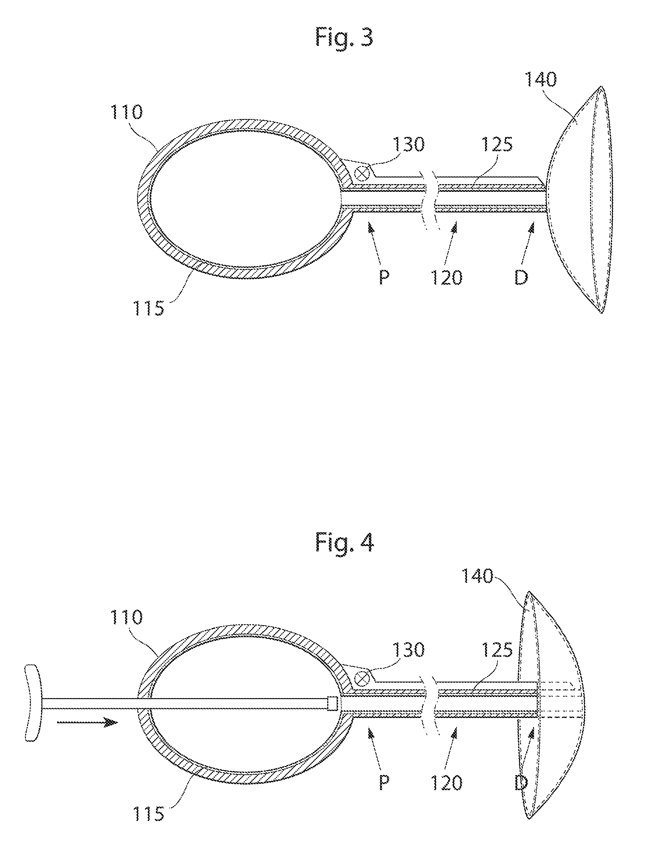

Figure 3 schematically illustrates how a mould member 140 adapted to a convex joint surface is connected to the proposed apparatus according to one embodiment.

In addition to the embodiment shown in Figure 1, the apparatus of Figure 3 includes a flexible and collapsible mould member 140. This member 140 has a pre-produced shape adapted to a shape and size of at least one damaged surface of a specific joint, for instance the surface S of the femoral head represented in Figure 2. The shape and size of the at least one damaged surface may be determined via a magnetic resonance imaging investigation, a computer tomography x-ray investigation and/or via arthroscopy. The pre-produced shape of the mould member 140 renders it adapted to contact and cover the joint surface S.

Moreover, the mould member 140 is configured to be inserted into the joint J in question and be form-fitted to the damaged surface S. Depending on the location and type of joint, fitting the mould member 140 to the surface S may require a number of additional instruments (not shown). In any case, the mould member 140 is configured to be connected to the distal end D of the instrument, 120 and when placed in the joint J, receive the material in liquid form being fed through the instrument 120. The mould member 140 has an internal volume that is configured to be filled with liquid material received via the instrument 120. Furthermore, after that the mould member 140 has been filled with the liquid biocompatible material, the mould member 140 is configured to enable the predefined conditions, which are required to accomplish a transition of the biocompatible material from the liquid form to the solid form.

Analogous to the embodiments described above with reference to Figure 1, the apparatus preferable includes a light source 130, e.g. arranged in the instrument 120, configured to illuminate the joint being treated during deposition of the liquid material in the mould member 140.

It is further advantageous if an arthroscopic instrument being inserted into the joint along with the instrument 120 includes a camera for viewing the joint.

Equivalent to the instrument 120 as such, the mould member 140 is configured to be inserted into the joint J by passing via a bone 210 or 220 of the body. This may involve passing via a bone of the body from inside the abdomen, or passing via the femoral bone of the body.

According to some embodiments, the mould member 140 is preferably adapted to be withdrawn (at least partly) from the joint J after that the biocompatible material has assumed its solid form.

Figure 4 schematically illustrates how a mould member 140 having a pre-produced shape adapted to a concave joint surface is connected to the proposed apparatus according to one embodiment. Here, all reference signs which also occur in Figure 1 or 3 designate the same components/features as those described above with reference to these figures.

According to one embodiment, the reservoir 110 is configured to hold the material at an elevated pressure level exceeding the normal atmospheric level. Thus, the mould member 140 may expand in response to receiving the material. Naturally, this is applicable to any configuration of the proposed mould member (i.e. not just the specific design shown in Figure 4).

Additionally, the apparatus may include an injection member 150 configured to inject the liquid material into the mould member 140 at the elevated pressure. Specifically, the material is injected into the mould member 140 through the instrument 120. When the mould member 140 has been filled with liquid material, this material is caused to transition from the liquid form to the solid form.

Figure 5 illustrates how the knee joint J of a patient may be treated according to one embodiment. Here, a mould member 140 is form-fitted to one of the joint surfaces of the femur bone facing the tibia bone 510, for instance by means of the apparatus shown in Figure 3.

Preferably, in this case, the distal end D of the instrument 120 is configured to be inserted into the joint J by passing via a capsula of the joint J.

Fig. 6 shows a lateral view of the body of a human patient, with the hip joint shown in section. The hip joint comprises a caput femur 5 placed at the very top of collum femur 6 which is the top part of the femur bone 7. The caput femur 5 is in connection with the acetabulum 8 which is a bowl shaped part of the pelvic bone 9. Laparoscopic/Arthroscopic trocars 33a,b,c is being used to reach the hip joint 39 with one or more camera 34, a surgical instrument 35 adapted to create a hole in the pelvic bone 9, or instruments 36 for introducing, placing, connecting, attaching, creating or filling a mould or an injected fluid.

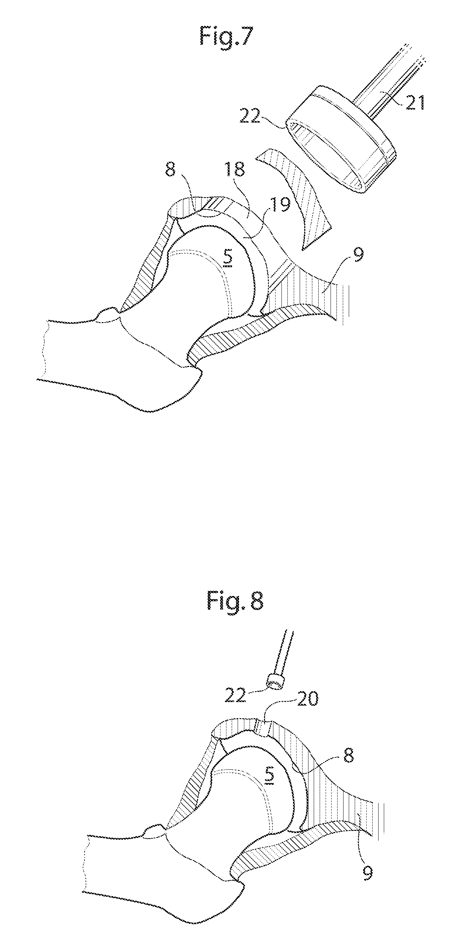

Fig. 7 shows an embodiment, wherein the mould is to be used for resurfacing the hip joint. For placing the mould in the hip joint the hip joint needs to be reached, this could be through a hole placed in the pelvic bone 9, the femoral bone 7 or the hip joint capsule 12. Fig. 7 shows the hole 18 in the pelvic bone 9 according to a first embodiment, the hole 18 is large which allows the mould to pass through said hole 18 in its full functional size.

Fig. 8 shows the hole 20 according to a second embodiment wherein the hole 20 created in a surgical or laparoscopic method is much smaller allowing the surgical instrument creating the hole to be smaller, and thus the incision and dissection performed in the human body. To place the mould in the joint in this embodiment the mould needs to be flexible or collapsible.

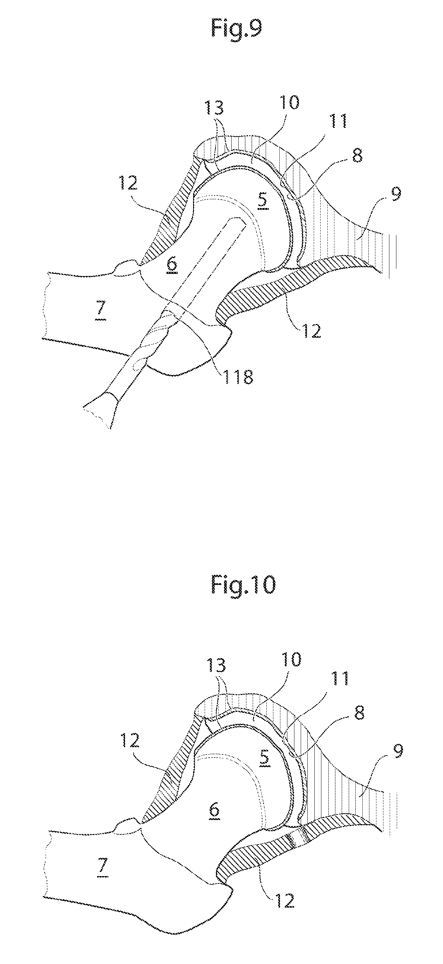

Fig. 9 shows the hip joint in section when creating a hole in the femur bone 7. The hole in the femur bone passes through the caput femur 5 into the hip joint and enables the surgeon to reach the hip joint.

Fig. 10 shows the hip joint in section when creating a hole in the hip joint capsule 12. The hole in the hip joint capsule passes into the hip joint and enables the surgeon to reach the hip joint.

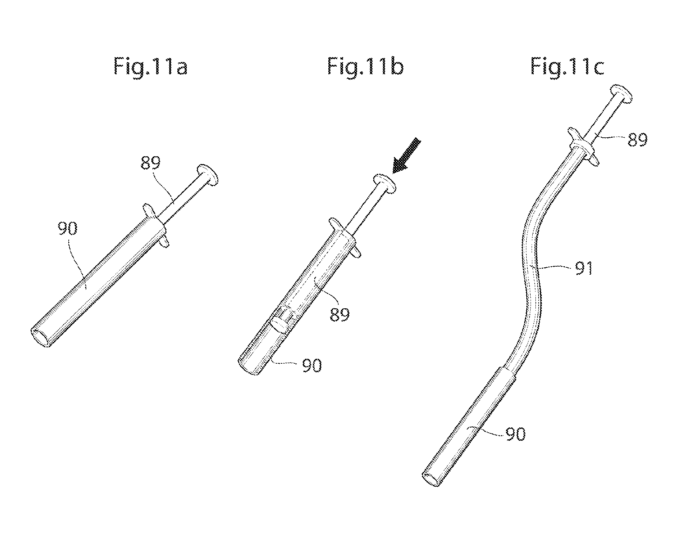

Fig. 11a shows an instrument for placing a mould 81 in the hip joint or the knee joint through a hole in the pelvic bone, the femur bone, the hip joint capsule or an area of the knee. The instrument comprises a piston 89 for transporting the mould 81 into the joint.

Fig. 11b shows a section of the surgical instrument comprising a tube like element 90 for housing of said mould 81.

Fig 11c shows the surgical instrument according to another embodiment in which the surgical instrument comprises a flexible or bent part 91 improving the reach of the surgical instrument. The surgical instrument according to any of the embodiments can be used to place said mould 81 inside of a joint in any of the ways described in the following embodiments.

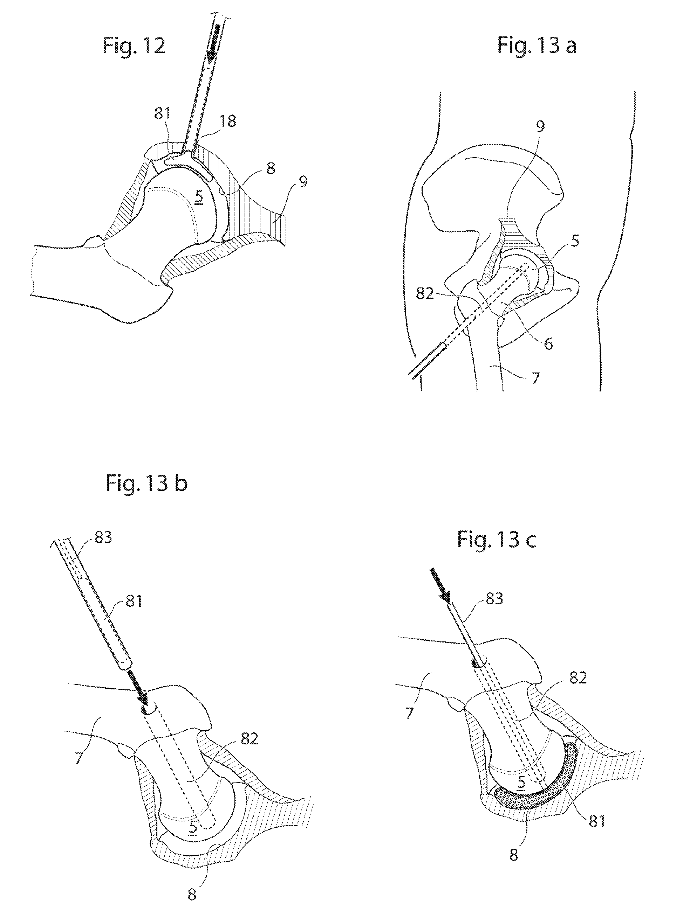

Fig. 12 shows the step of placing a mould 81 inside of the hip joint of a human patient through a hole 18 in the pelvic bone 9. The step of placing said mould 81 can be performed in a surgical, or in a laparoscopic/arthroscopic method.

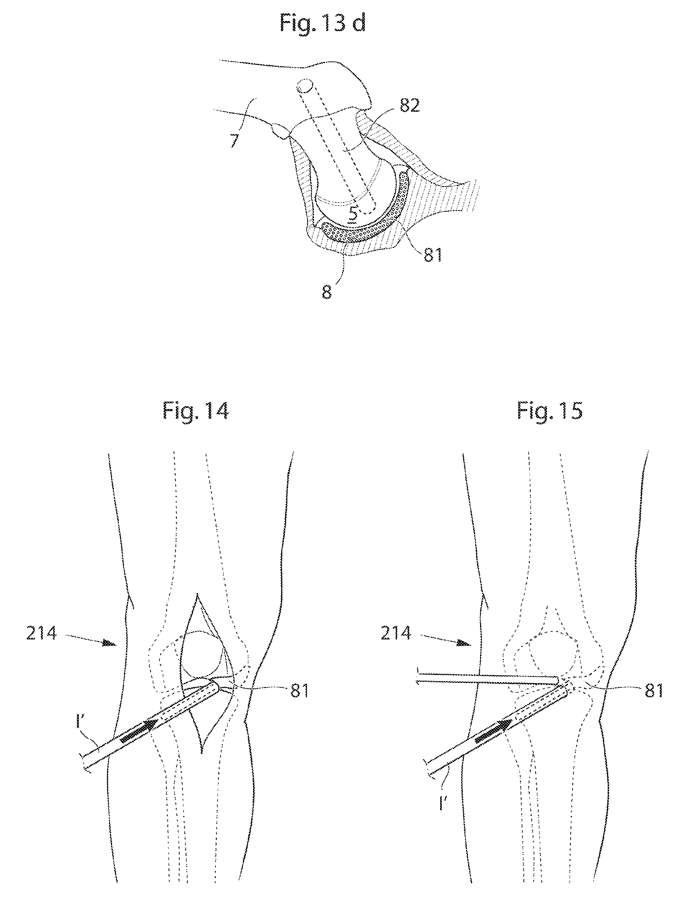

Fig. 13a,b,c,d shows an alternative approach to placing said mould 81 in the hip joint of a human patient. Said alternative approach comprises the steps of creating a hole 82 in the femur bone 7 following a length axis of the collum femur 6, said hole starting from the lateral side of the thigh, penetrating the cortex of the femur bone 7 and eventually reaching the cortex of the caput femur 5 from the inside thereof, penetrating said cortex and entering into the hip joint. After the creation of the hole 82 in the femur bone 7 the mould 81 is inserted into the hip joint through the hole 82 using the surgical instrument 83 according to any of the embodiments above, as shown in fig. 13b.

Fig 13c shows the mould 81 when being inserted into the hip joint using the surgical instrument 83 adapted therefore.

Fig. 13d shows the mould 81 in place after insertion into the hip joint, the surgical instrument used to place said mould 81 in the hip joint is retracted after the insertion is completed.

Fig. 14 shows the placing of a mould 81 in a knee 214 in a surgical method. The mould 81 is placed using the surgical instrument according to any of the embodiments above.

Fig. 15 shows the placing of a mould 81 in a knee 214 in a laparoscopic/arthroscopic method. The mould 81 is placed using the surgical instrument according to any of the embodiments above.

After the mould has been placed in the hip or knee joint it is filled with a fluid adapted to harden to a medical device adapted to serve as at least one artificial joint surface.

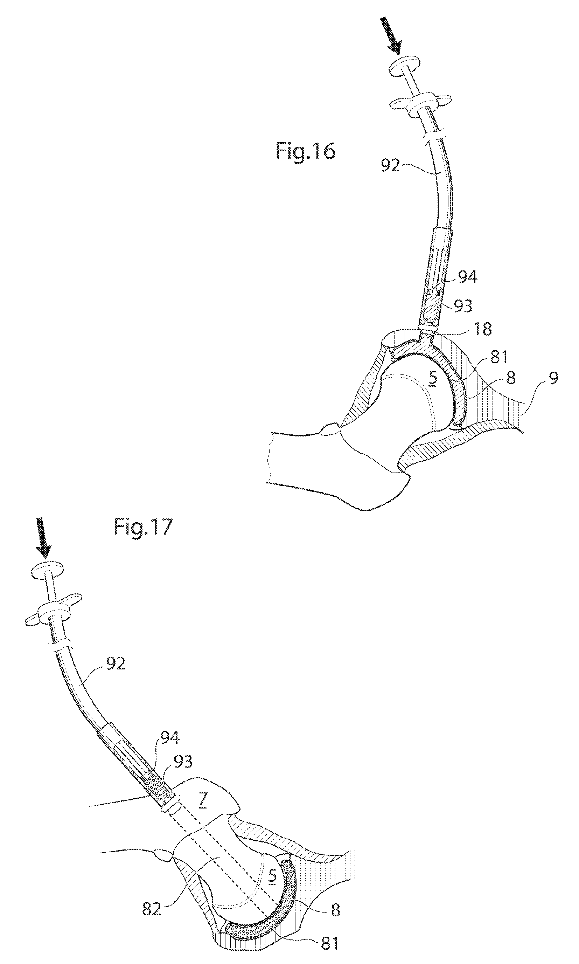

Fig. 16 shows the hip joint in section wherein an injecting member 92 injects a fluid 93 into a mould 81 in the hip joint through a hole 18 in the pelvic bone 9 from the opposite side from acetabulum 8. The injecting member 92 comprises a piston 94 that pushes said fluid 93 into the mould 81.

Fig. 17 shows the hip joint in section wherein an injecting member 92 injects a fluid 93 into a mould 81 in the hip joint through a hole 82 in the femur bone 7. The injecting member 92 comprises a piston 94 that pushes said fluid 93 into the mould 81.

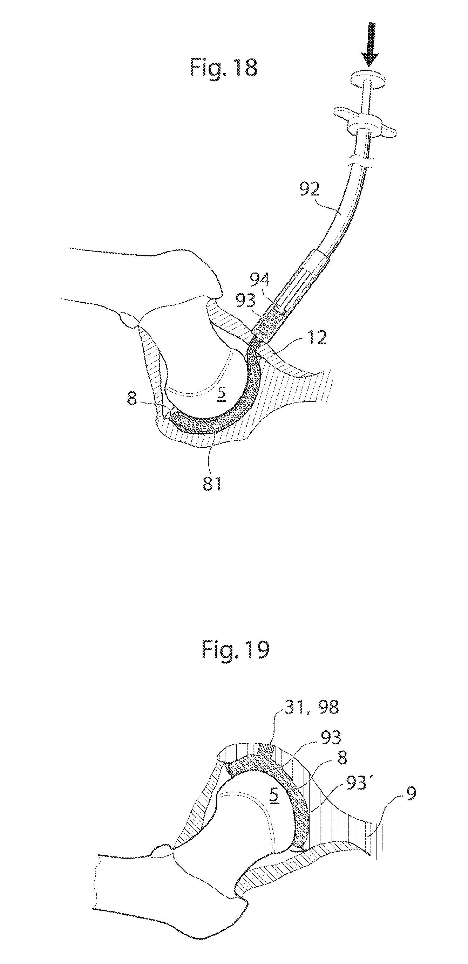

Fig. 18 shows the hip joint in section, wherein an injecting member injects a fluid 93 into a mould 81 in the hip joint through a hole in the hip joint capsule 12. The injecting member 92 comprises a piston 94 that pushes said fluid 93 into the mould 81. The fluid 93 is adapted to harden to create a medical device adapted to serve as at least one artificial hip joint surface.

Fig. 19 shows the hip joint in section wherein the medical device 93' is located between the acetabulum 8 and the caput femur 5 which has been created by the hardening of the fluid 93 adapted to harden. Said medical device is adapted to serve as at least one artificial hip joint surface. The hole in the pelvic bone is preferably sealed with a bone plug 31 or a prosthetic part 98. The mould 81 used to create the medical device 93' has been removed.

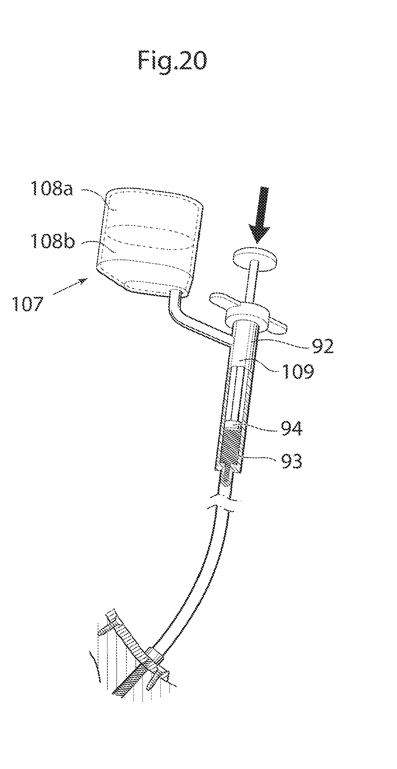

Fig. 20 shows the injecting member 92 according to any of the embodiments above, adapted to inject fluid 93 into a mould 81 in the hip joint or the knee joint. The injecting member 92 could further be adapted to inject material 93 or a fluid 93 into a connecting area between the pelvic bone 9 and a prosthetic part, the pelvic bone 9 and a bone plug 31 or the caput femur 5 and a prosthetic part. Said injecting member 92 comprises a container 107 adapted to hold a fluid 93 for injection. According to a first embodiment said container 107 comprises two compartments 108a,b adapted to hold two different fluids, said fluids being adapted to harden when mixed. In the embodiment when the container 107 is adapted to hold two fluids, it is conceivable that the injecting member 105 further comprises a mixing member 109 wherein said two fluids are being mixed before injection. According to a second embodiment (not shown) said container 107 is adapted to keep said fluid sterile. According to a third embodiment (not shown) said container 107 is adapted to keep said fluid cold or hot and according to a fourth embodiment (not shown) said container 107 is adapted to keep said fluid in a dark environment. Furthermore a combination of the above mentioned embodiments is conceivable.

The term "comprises/comprising" when used in this specification is taken to specify the presence of stated features, integers, steps or components. However, the term does not preclude the presence or addition of one or more additional features, integers, steps or components or groups thereof.

The invention is not restricted to the described embodiments in the figures, but may be varied freely within the scope of the claims.

## Claims

1. A system for treatment of arthrosis in a joint of a mammal or human patient, by replacing damaged bone, the system comprising:
a biocompatible material for replacing damaged bone of a worn out joint surface, wherein the biocompatible material is configured to assume a solid form under predefined conditions, and when in the solid form having a resistance to wear,
a reservoir (110) configured to hold a volume of a biocompatible material in liquid form having a temperature higher than 100°C outside of a body containing a joint (J) to be treated, and
an instrument (120) having a general tube shape, a distal end (D) of the instrument (120) being configured to be inserted into the joint (J), a proximal end (P) of the instrument (120) being connected to the reservoir (110) and configured to receive the liquid material there from, wherein the instrument (120) is configured to feed the liquid material having a temperature higher than 100°C from the proximal end (P) to the distal end (D) for deposition on at least one damaged surface (S) of the joint (J), such that adjacent nerves are damaged by the heat of the material, thus preventing pain from the damaged surface.

2. The system according to claim 1, further comprising a flexible and collapsible mould member (140) having a pro-produced shape adapted to a shape and size of at least one of the at least one damaged surface (S) so as to contact and cover this surface (S), the mould member (140) being configured to be inserted into the joint (J) and be form-fitted to said damaged surface (S), be connected to the distal end (D) of the instrument (120) and when placed in the joint (J) receive the material in liquid form being fed through the instrument (120), the mould member (140) having an internal volume configured to be filled with liquid material received via the instrument (120).

3. The system according to claim 2, wherein the mould member (140) is configured to be at least partly withdrawn from the joint (J) after that the material has assumed its solid form.

4. The system according to anyone of claims 2 - 3, wherein the reservoir (110) is configured to hold the material at an elevated pressure level exceeding the normal atmospheric level, and the mould member (140) is configured to expand in response to receiving the material.

5. The system according to any one of claims 2 - 4, comprising an injection member (150) configured to inject the liquid material into the mould member (140) at an elevated pressure.

6. The system according to any one of claims 2 - 5, wherein at least one of;
a mould member (140), comprising an isolation material configured to reduce the risk of body tissue being damaged by the liquid material having a temperature above 37 degrees Celsius
a reservoir (110) comprising at least one shield member (115) configured to isolate the reservoir (110) from the body, and
an instrument comprising at least one shield member (125), configured to isolate the body from liquid material passing through the instrument (120).

7. The system according to any one of claims 1 - 6, wherein the reservoir (110) is configured to hold the biocompatible material at a temperature of at least one of; at least 300 degrees Celsius, and at least 400 degrees Celsius.

8. The system according to any one of claims 1 - 7, wherein said bio-compatible material comprises fluoropolymers, comprising at least one of one of polytetrafluoroethylene, perfluoroalkoxy or fluorinated ethylene propylene.

9. The system according to any one of claims 1 - 8, wherein the biocompatible material comprises two components, each component when isolated from the other component being a liquid, the components when mixed in predefined proportions developing a solid material, the reservoir (110) being configured to:
hold the two components separated from one another during an initial phase of a treatment, and
mix the two components into a mix during a surgery phase subsequent to the initial phase, and wherein the instrument (120) being configured to feed the mix to the at least one damaged joint surface (S).

10. The system according to any one of claims 1 - 9, wherein the biocompatible material is configured to at least one of:
be liquid when an exposure to electromagnetic radiation in a predefined spectrum is below a first predefined energy level per unit volume, and
be solid when the exposure to electromagnetic radiation in the predefined spectrum is above a second predefined energy level per unit volume, wherein the apparatus comprising an electromagnetic radiation source configured to convey electromagnetic radiation in the predefined spectrum to the at least one damaged joint surface (S) via a instrument (120).

11. The system according to any one of claims 1 - 9, wherein the biocompatible material is configured to:
be liquid when an exposure to ultrasonic energy in a predefined spectrum is below a primary predefined energy level per unit volume, and
be solid when the exposure to ultrasonic energy in the predefined spectrum is above a secondary predefined energy level per unit volume, wherein
the apparatus comprising an ultrasound source configured to convey ultrasonic energy in the predefined spectrum to the at least one damaged joint surface (S) via a instrument (120).

12. The system according to claims 1 - 11, wherein the reservoir (110) and a connection of the reservoir (110) towards the instrument (120) are configured to maintain the biocompatible material sterile.

13. The system according to any one of claims 1 - 12, wherein the distal end (D) of the instrument (120) is configured to enable insertion into a hip joint (J) by passing via a bone (220) of the body from inside the abdomen.

14. The system according to any one of the proceeding claims, comprising a light source (130) configured to illuminate the joint (J) during deposition of the liquid material on the at least one damaged surface (S).

15. The system according to any one of claims 1 - 14, wherein said material comprises at least one material selected from the group consisting of:
polytetrafluoroethylene,
perfluoroalkoxy
fluorinated ethylene propylene,
polyethylene, and
acrylic polymer mixed with alumina trihydrate.

## Patentansprüche

1. System zum Behandeln von Arthrose in einem Gelenk eines Säuger- oder menschlichen Patienten durch Ersetzen von geschädigtem Knochen, wobei das System umfasst:
ein biokompatibles Material zum Ersetzen von geschädigtem Knochen einer abgenutzten Gelenkoberfläche, wobei das biokompatible Material dafür gestaltet ist, unter vorbestimmten Bedingungen eine feste Form anzunehmen und, wenn in der festen Form, Verschleißfestigkeit aufzuweisen,
ein Reservoir (110), das zum Halten eines Volumens eines biokompatiblen Materials in flüssiger Form mit einer Temperatur von höher als 100 °C außerhalb eines Körpers, der ein zu behandelndes Gelenk (J) enthält, gestaltet ist, und
ein Instrument (120) mit einer allgemein rohrförmigen Form, wobei ein distales Ende (D) des Instruments (120) dafür gestaltet ist, in das Gelenk (J) eingeführt zu werden, ein proximales Ende (P) des Instruments (120) mit dem Reservoir (110) verbunden ist und dafür gestaltet ist, das flüssige Material daraus aufzunehmen, wobei das Instrument (120) dafür gestaltet ist, das flüssige Material mit einer Temperatur von höher als 100 °C von dem proximalen Ende (P) zu dem distalen Ende (D) zum Aufbringen auf wenigstens eine geschädigte Oberfläche (S) des Gelenks (J) zu befördern, so dass benachbarte Nerven durch die Hitze des Materials geschädigt werden und so Schmerz von der geschädigten Oberfläche verhindert wird.

2. System gemäß Anspruch 1, ferner umfassend ein flexibles und zusammenfaltbares Gussformelement (140) mit einer vorhergestellten Form, die einer Form und Größe von wenigstens einer der wenigstens einen geschädigten Oberfläche (S) angepasst ist, um mit dieser Oberfläche (S) in Kontakt zu kommen und sie zu bedecken, wobei das Gussformelement (140) dafür gestaltet ist, in das Gelenk (J) eingeführt zu werden und formschlüssig mit der geschädigten Oberfläche (S) gemacht zu werden, mit dem distalen Ende (D) des Instruments (120) verbunden zu werden und, wenn in dem Gelenk (J) platziert, das Material in flüssiger Form aufzunehmen, das durch das Instrument (120) befördert wird, wobei das Gussformelement (140) ein Innenvolumen aufweist, das dafür gestaltet ist, mit dem über das Instrument (120) erhaltenen Material gefüllt zu werden.

3. System gemäß Anspruch 2, wobei das Gussformelement (140) dafür gestaltet ist, wenigstens teilweise aus dem Gelenk (J) zurückgezogen zu werden, nachdem das Material seine feste Form angenommen hat.

4. System gemäß einem der Ansprüche 2-3, wobei das Reservoir (110) dafür gestaltet ist, das Material bei einem erhöhten Druck zu halten, der normalen Atmosphärendruck übersteigt, und das Gussformelement (140) dafür gestaltet ist, in Antwort auf das Aufnehmen des Materials zu expandieren.

5. System gemäß einem der Ansprüche 2-4, umfassend ein Injektionselement (150), das dafür gestaltet ist, das flüssige Material mit einem erhöhten Druck in das Gussformelement (140) einzuspritzen.

6. System gemäß einem der Ansprüche 2-5, wobei wenigstens eines von:
ein Gussformelement (140) umfassend ein Isolationsmaterial, das zum Verringern des Risikos gestaltet ist, dass Körpergewebe durch das flüssige Material mit einer Temperatur von über 37 Grad Celsius geschädigt wird,
ein Reservoir (110) umfassend wenigstens ein Abschirmelement (115), das zum Isolieren des Reservoirs (110) von dem Körper gestaltet ist, und
ein Instrument umfassend wenigstens ein Abschirmelement (125), das zum Isolieren des Körpers von flüssigem Material, das durch das Instrument (120) fließt, gestaltet ist.

7. System gemäß einem der Ansprüche 1-6, wobei das Reservoir (110) zum Halten des biokompatiblen Materials bei einer Temperatur von wenigstens einem von wenigstens 300 Grad Celsius und wenigstens 400 Grad Celsius gestaltet ist.

8. System gemäß einem der Ansprüche 1-7, wobei das biokompatible Material Fluorpolymere umfassend wenigstens eines von Polytetrafluorethylen, Perfluoralkoxy oder fluoriertes Ethylenpropylen umfasst.

9. System gemäß einem der Ansprüche 1-8, wobei das biokompatible Material zwei Komponenten umfasst, wobei jede Komponente, wenn von der anderen Komponente isoliert, eine Flüssigkeit ist, wobei die Komponenten, wenn in vorbestimmten Verhältnissen gemischt, ein festes Material entwickeln, wobei das Reservoir (110) dafür gestaltet ist:
während einer Anfangsphase der Behandlung die beiden Komponenten getrennt voneinander zu halten und
während einer chirurgischen Phase nach der Anfangsphase die beiden Komponenten zu einem Gemisch zu mischen und wobei
das Instrument (120) dafür gestaltet ist, das Gemisch der wenigstens einen geschädigten Gelenkoberfläche (S) zuzuführen.

10. System gemäß einem der Ansprüche 1-9, wobei das biokompatible Material dafür gestaltet ist, wenigstens eines von:
flüssig zu sein, wenn eine Exposition gegenüber elektromagnetischer Strahlung in einem vorbestimmten Spektrum unter einer ersten vorbestimmten Energiemenge pro Volumeneinheit liegt, und
fest zu sein, wenn die Exposition gegenüber elektromagnetischer Strahlung in dem vorbestimmten Spektrum über einer zweiten vorbestimmten Energiemenge pro Volumeneinheit liegt, wobei
die Vorrichtung eine Quelle von elektromagnetischer Strahlung umfasst, die dafür gestaltet ist, über ein Instrument (120) elektromagnetische Strahlungen in dem vorbestimmten Spektrum an die wenigstens eine geschädigte Gelenkoberfläche (S) abzugeben.

11. System gemäß einem der Ansprüche 1-9, wobei das biokompatible Material dafür gestaltet ist:
flüssig zu sein, wenn eine Exposition gegenüber Ultraschallenergie in einem vorbestimmten Spektrum unter einer primären vorbestimmten Energiemenge pro Volumeneinheit liegt, und
fest zu sein, wenn die Exposition gegenüber Ultraschallenergie in dem vorbestimmten Spektrum über einer sekundären vorbestimmten Energiemenge pro Volumeneinheit liegt, wobei
die Vorrichtung eine Ultraschallquelle umfasst, die dafür gestaltet ist, über ein Instrument (120) Ultraschallenergie in dem vorbestimmten Spektrum an die wenigstens eine geschädigte Gelenkoberfläche (S) abzugeben.

12. System gemäß Ansprüchen 1-11, wobei das Reservoir (110) und eine Verbindung des Reservoirs (110) zu dem Instrument (120) dafür gestaltet sind, das biokompatible Material steril zu halten.

13. System gemäß einem der Ansprüche 1-12, wobei das distale Ende (D) des Instruments (120) dafür gestaltet ist, das Einführen in ein Hüftgelenk (J) durch Durchtreten durch einen Knochen (220) des Körpers von innerhalb des Abdomens zu erlauben.

14. System gemäß einem der vorstehenden Ansprüche, umfassend eine Lichtquelle (130), die dafür gestaltet ist, das Gelenk (J) während des Aufbringens des flüssigen Materials auf die wenigstens eine geschädigte Oberfläche (S) zu beleuchten.

15. System gemäß einem der Ansprüche 1-14, wobei das Material wenigstens ein Material ausgewählt aus der Gruppe bestehend aus:
Polytetrafluorethylen,
Perfluoralkoxy,
fluoriertes Ethylenpropylen,
Polyethylen und
Acrylpolymer gemischt mit Aluminiumoxidtrihydrat
umfasst.

## Revendications

1. Système pour le traitement de l'arthrose dans une articulation d'un patient mammifère ou humain, par remplacement d'os endommagé, le système comprenant :
un matériau biocompatible pour remplacer de l'os endommagé d'une surface articulaire usée, le matériau biocompatible étant configuré pour prendre une forme solide dans des conditions prédéfinies, et lorsqu'il est sous la forme solide, ayant une résistance à l'usure,
un réservoir (110) configuré pour contenir un volume d'un matériau biocompatible sous forme liquide ayant une température supérieure à 100 °C à l'extérieur d'un corps contenant une articulation (J) à traiter, et
un instrument (120) ayant une forme générale de tube, une extrémité distale (D) de l'instrument (120) étant configurée pour être inséré dans l'articulation (J), une extrémité proximale (P) de l'instrument (120) étant raccordée au réservoir (110) et configurée pour recevoir le matériau liquide depuis celui-ci, l'instrument (120) étant configuré pour alimenter le matériau liquide ayant une température supérieure à 100 °C de l'extrémité proximale (P) vers l'extrémité distale (D) pour dépôt sur au moins une surface endommagée (S) de l'articulation (J), de sorte que les nerfs adjacents soient endommagés par la chaleur du matériau, de manière à prévenir la douleur depuis la surface endommagée.

2. Système selon la revendication 1, comprenant en outre un élément de moule flexible et pliable (140) ayant une forme pré-produite adaptée à une forme et une taille d'au moins un de l'au moins une surface endommagée (S) de manière à entrer en contact avec et recouvrir cette surface (S), l'élément de moule (140) étant configuré pour être inséré dans l'articulation (J) et être ajusté en forme à la dite surface endommagée (S), être raccordé à l'extrémité distale (D) de l'instrument (120) et lorsqu'il est placé dans l'articulation (J), recevoir le matériau sous forme liquide étant alimenté par l'intermédiaire de l'instrument (120), l'élément de moule (140) ayant un volume interne configuré pour être rempli de matériau liquide reçu par l'intermédiaire de l'instrument (120).

3. Système selon la revendication 2, dans lequel l'élément de moule (140) est configuré pour être au moins partiellement retiré de l'articulation (J) une fois que le matériau a pris sa forme solide.

4. Système selon l'une quelconque des revendications 2 à 3, dans lequel le réservoir (110) est configuré pour contenir le matériau à un niveau de pression élevé dépassant le niveau atmosphérique normal, et l'élément de moule (140) est configuré pour se dilater en réponse à la réception du matériau.

5. Système selon l'une quelconque des revendications 2 à 4, comprenant un élément d'injection (150) configuré pour injecter le matériau liquide dans l'élément de moule (140) à une pression élevée.

6. Système selon l'une quelconque des revendications 2 à 5, dans lequel au moins l'un de :
un élément de moule (140), comprenant un matériau d'isolation configuré pour réduire le risque qu'un tissu corporel soit endommagé par le matériau liquide ayant une température supérieure à 37 degrés Celsius un réservoir (110) comprenant au moins un élément de protection (115) configuré pour isoler le réservoir (110) du corps, et
un instrument comprenant au moins un élément de protection (125), configuré pour isoler le corps du matériau liquide traversant l'instrument (120).

7. Système selon l'une quelconque des revendications 1 à 6, dans lequel le réservoir (110) est configuré pour contenir le matériau biocompatible à une température d'au moins l'un de : au moins 300 degrés Celsius, et au moins 400 degrés Celsius.

8. Système selon l'une quelconque des revendications 1 à 7, dans lequel ledit matériau biocompatible comprend des fluoropolymères, comprenant au moins l'un parmi un polytétrafluoroéthylène, un perfluoroalcoxy ou éthylène-propylène fluoré.

9. Système selon l'une quelconque des revendications 1 à 8, dans lequel le matériau biocompatible comprend deux composants, chaque composant, lorsqu'il est isolé de l'autre composant, étant un liquide, les composants, lorsqu'ils sont mélangés dans des proportions prédéfinies, formant un matériau solide, le réservoir (110) étant configuré pour :
contenir les deux composants séparés l'un de l'autre pendant une phase initiale d'un traitement, et
mélanger les deux composants dans un mélange pendant une phase de chirurgie consécutive à la phase initiale, et dans lequel
l'instrument (120) étant configuré pour alimenter le mélange sur l'au moins une surface articulaire endommagée (S).

10. Système selon l'une quelconque des revendications 1 à 9, dans lequel le matériau biocompatible est configuré pour au moins l'un de :
être liquide lorsqu'une exposition à un rayonnement électromagnétique dans un spectre prédéfini est inférieure à un premier niveau d'énergie prédéfini par unité de volume, et
être solide lorsque l'exposition à un rayonnement électromagnétique dans le spectre prédéfini est supérieure à un deuxième niveau d'énergie prédéfini par unité de volume, dans lequel
l'appareil comprenant une source de rayonnement électromagnétique configurée pour appliquer un rayonnement électromagnétique dans le spectre prédéfini à l'au moins une surface articulaire endommagée (S) par l'intermédiaire d'un instrument (120).

11. Système selon l'une quelconque des revendications 1 à 9, dans lequel le matériau biocompatible est configuré pour :
être liquide lorsqu'une exposition à une énergie ultrasonore dans un spectre prédéfini est inférieure à un niveau d'énergie prédéfinie primaire par unité de volume, et
être solide lorsque l'exposition à une énergie ultrasonore dans le spectre prédéfini est supérieure à un niveau d'énergie prédéfini secondaire par unité de volume, dans lequel
l'appareil comprenant une source d'ultrasons configurée pour appliquer une énergie ultrasonore dans le spectre prédéfini à l'au moins une surface articulaire endommagée (S) par l'intermédiaire d'un instrument (120).

12. Système selon les revendications 1 à 11, dans lequel le réservoir (110) et un raccordement du réservoir (110) à l'instrument (120) sont configurés pour maintenir le matériau biocompatible stérile.

13. Système selon l'une quelconque des revendications 1 à 12, dans lequel l'extrémité distale (D) de l'instrument (120) est configurée pour permettre l'insertion dans une articulation de la hanche (J) par passage par l'intermédiaire d'un os (220) du corps depuis l'intérieur de l'abdomen.

14. Système selon l'une quelconque des revendications précédentes, comprenant une source de lumière (130) configurée pour illuminer l'articulation (J) pendant le dépôt du matériau liquide sur l'au moins un surface endommagée (S).

15. Système selon l'une quelconque des revendications 1 à 14, dans lequel ledit matériau comprend au moins un matériau choisi dans le groupe constitué de :
polytétrafluoroéthylène,
perfluoroalcoxy
éthylène-propylène fluoré,
polyéthylène, et
polymère acrylique mélangé avec de l'alumine trihydratée.
